Europäisches Patentamt

European Patent Office

Office européen des brevets

(11) Veröffentlichungsnummer: **0 322 712 B1**

(19)

(12) **EUROPÄISCHE PATENTSCHRIFT**

(45) Veröffentlichungstag der Patentschrift: **25.11.92**

(21) Anmeldenummer: **88121299.7**

(22) Anmeldetag: **20.12.88**

(51) Int. Cl.⁵: **A61K 39/015**, C12N 15/30,
C12P 21/02, C12N 15/62,
C12Q 1/68, G01N 33/569

(54) **Malaria-spezifische DNA-Sequenzen, ihre Expressionsprodukte und deren Verwendung.**

(30) Priorität: **30.12.87 DE 3744495**
**15.09.88 DE 3831351**

(43) Veröffentlichungstag der Anmeldung:
**05.07.89 Patentblatt 89/27**

(45) Bekanntmachung des Hinweises auf die
Patenterteilung:
**25.11.92 Patentblatt 92/48**

(84) Benannte Vertragsstaaten:
**AT BE CH DE ES FR GB GR IT LI LU NL SE**

(56) Entgegenhaltungen:
**EP-A- 0 309 746**

**THE JOURNAL OF CLINICAL INVESTIGATION,
Band 75, Mai 1985, Seiten 1718-1725; L.H.
PERRIN et al.: "Immunization with a plasmodium falciparum merozoite surface antigen
induces a partial immunity in monkeys"**

(73) Patentinhaber: **BEHRINGWERKE Aktiengesellschaft
Postfach 1140
W-3550 Marburg 1(DE)**

(72) Erfinder: **Knapp, Bernhard, Dr.
Baumgarten 9
W-3550 Marburg-Schröck(DE)**
Erfinder: **Hundt, Erika, Dr.
Zum Hirtzborn 8
W-3550 Marburg-Wehrshausen(DE)**
Erfinder: **Enders, Burkhard, Dr.
Oberer Eichweg 12
W-3550 Marburg(DE)**
Erfinder: **Küpper, Hans, Dr.
Biegenstrasse 39
W-3550 Marburg(DE)**

(74) Vertreter: **Fischer, Hans-Jürgen, Dr. et al
Hoechst AG Zentrale Patentabteilung Postfach 80 03 20
W-6230 Frankfurt am Main 80(DE)**

**PROCEEDINGS OF THE NATIONAL ACADEMY OF SCIENCES OF THE USA, Band 81, Juni 1984, Seiten 3690-3694; M.J. McGARVEY et al.: "Identification and expression in Escherichia coli of merozoite stage-specific genes of the human malarial parasite Plasmodium falciparum"**

**Beschreibung**

Ein wichtiger Schritt zur Entwicklung einer Vaccine gegen Malaria ist die Identifizierung protektiver Antigene. Als protektiv werden im allgemeinen solche Antigene eingestuft, die bei in vivo Versuchen im Tiermodell wie z.B. in Saimiri- oder Aotus-Affen einem Schutz vor einer intravenös gesetzten P.falciparum-Infektion ergeben haben. Bisher sind nur unbefriedigende Schutzversuche am Menschen beschrieben worden, aber mehrere isolierte P.falciparum Proteine haben eine komplette oder teilweise Schutzwirkung im Tiermodell gezeigt. Dies trifft sowohl für gelelektrophoretisch gereinigte Proteinfraktionen von 75kD und 100kD zu als auch für die gelelektrophoretisch gereinigten Proteinbanden der Molekulargewichte 200kD, 140kD und 41kD (L.H. Perrin et al. (1984), Clin.exp.Immunol. 56, 67-72; L.H. Perrin et al. (1985) J.Clin.Invest. 75, 1718-1721; W.A. Siddiqui et al. (1987), Proc.Natl. Acad.Sci., USA 84, 3014-3018). Von den bisher biotechno-logisch hergestellten für Merozoiten spezifischen Proteinen zeigten ein rekombinantes Expressionsprotein der "5′ repeat Region" des sog. RESA 155kD Merozoitenproteins als auch ein synthetisches Oligopeptid des 200kD Merozoitoberflächen-Vorläuferproteins sowie eine Kombination synthetischer Oligopeptide von Proteinen der Molekulargewichte 35kD, 55kD und 200kD in Immunisierungsversuchen mit Saimiri- oder Aotusaffen eine partielle Schutzwirkung. Gentechnologisch hergestellte rekombinante Proteine der obigen Antigene, welche eine Schutzwirkung in in vivo Experimenten mit Affen zeigen, sind potentielle Kandidaten für eine Malaria-Vaccine.

Ziel der Arbeiten war es, codierende Sequenzen für die von L. Perrin (1985 a.a.O) beschriebene protektive 41kD-Antigenbande zu isolieren, die Sequenzen zur Expression zu bringen und die Expressionsprodukte auf ihre protektive Wirkung hin im Affenmodell zu testen. Mit Hilfe eines spezifischen Antiserums gegen die 41 KD-Antigenbande wurden aus einer genomischen Expressionsbank fünfzehn Klone isoliert und die Struktur ihrer Insertionen aufgeklärt. Die Sequenzen der Klone 41-1 bis 41-10 und 41-12 bis 41-15, sowie 41-17 sind in Tab. 1-15 dargestellt. Mit Hilfe zweier immunologisch sehr intensiv reagierender Klone (41-2 und 41-7) wurden aus dem zum Screening verwendeten Serum monospezifische Antikörper isoliert. Diese Antikörper reagieren im Western Blot spezifisch mit einem Merozoiten-Antigen von 41 kD.

Mit der Insert DNA des Klons 41-2 hybridisierte im Southern Blot ein 3,0 kb EcoRI-Fragment und ein 2,0 kb Sau3A-Fragment. Beide DNA-Fragmente wurden isoliert und sequenziert.

Das Sau3A-Fragment enthält die vollständige codierende Region des 41-2 Gens. Diese enthält keine Introns und codiert für 184 Aminosäuren mit einem Molekulargewicht von 21512 D. Das 41-2 Protein besitzt eine Signalsequenz und zwei weitere hydrophobe Abschnitte. Repetitive Sequenzanteile sind nicht vorhanden. Western Blot Analyse von Schizontproteinen mit Kaninchen-Antiseren, welche gegen ein Expressionsprodukt hergestellt wurden, das 70 % der codierenden Region beinhaltet, ergab eine Bande von 29 kD. Weiterhin konnte durch Northern Blot Analyse eine mRNA von 1.6 kb nachgewiesen werden.

Die Insert DNA des Klons 41-7 codiert dagegen für das 41kD Protein. Kaninchen-Antiseren, welche gegen ein Fusionsprotein von 41-7 hergestellt wurden, erkennen im Western Blot eindeutig eine 41kD Bande. Durch Screening einer genomischen lambda gtll EcoRI* Genbank mit der Insert DNA des Klons 41-7 konnte ein Klon identifiziert werden, der ein malariaspezifisches Insert von 2.3kb enthält. Dieses wurde isoliert und sequenziert. Es enthält die gesamte codierende Region für ein 41kD Protein. Das Gen codiert keine Signalsequenz und enthält weder Introns noch repetitive Abschnitte. Die abgeleitete Aminosäuresequenz des 41kD Proteins von P.falciparum ist hochhomolog (>60%) zu Aldolasen aus Muskel und Leber von Säugern und zur Aldolase von Trypanosoma brucei. Im Gegensatz zum Säugergenom konnte für P.falciparum nur ein Aldolasegen pro Genom durch Southern Blot Analysen festgestellt werden.

Die Klone 41-1, 2, 3, 4, 5, 6, 7, 8, 9, 10, 12, 13, 14, 15, 17 wurden aufgrund ihrer Kreuzreaktivitäten mit dem Antiserum gegen die 41kD Proteinbande detektiert. Sie sind zur Herstellung einer Vaccine geeignet. Die Insert-DNAs der Klone 41-1 bis 41-5, sowie 41-7, 41-10 und 41-14 wurden in dem Vektor pEX-31 zur Expression gebracht und die resultierenden Fusionsproteine wurden gereinigt. Eine Kombination einer immunologisch wirksamen Menge von drei Expressionsprodukten (41-1, 41-2, 41-3) schützt Aotusaffen vor einer P.falciparum Infektion.

Die Erfindung betrifft folglich
a) die gereinigten und isolierten DNA-Sequenzen der Klone 41-1 bis 41-10, 41-12 bis 41-15 und 41-17 sowie 41-2gen einschließlich ihrer Transkriptionsprodukte,
b) diese Sequenzen ganz oder teilweise enthaltenden DNA-Strukturen und Vektoren,
c) mit solcher DNA transformierte pro- oder eukaryotische Zellen,
d) die von diesen Zellen auf Grund der Transformation exprimierten Polypeptide oder Teile davon,
e) deren Aminosäuresequenzen,
f) Antikörper gegen die Polypeptide unter (d) einschließlich ihrer Anwendung zur passiven Immunisierung, zur Diagnostik und zur Reinigung besagter Polypeptide,

g) Impfstoffe gegen Malaria, die Aminosäuresequenzen von (e) alleine oder in Kombination enthalten,

h) ein Verfahren zur gentechnischen Herstellung der unter (d) angeführten Polypeptide oder Teilen davon,

i) sowie Anwendung der besagten Aminosäuresquenzen zur Diagnostik.

Weitere Ausgestaltungen der Erfindung sind in den nachfolgenden Beispielen, Tabellen und den Patentansprüchen aufgeführt.

Beispiele

Beispiel 1:

Screening einer lambda gtII-Expressions-Bank mit dem monospezifischen anti-41kD Serum

$10^6$ PFU (plaque forming units) einer genomischen lambda gtII Expressionsbank (hergestellt aus DNA des P.falciparum-Stammes T996) wurden mit einem Antiserum gegen die 41kD Antigenbande (L.H. Perrin et al. (1985) a.a.O.) aus dem P.falciparum-Stamm SGE2 nach bekannten Methoden (L.S. Ozaki (1986), J. Immun. Method. 89, 213-219; Promega Biotec (1986), Proto Blot Immunoscreening System, Technical Manual) gescreent. Als Detektionssystem diente dabei ein anti-Kaninchen-IgG / alkalische Phosphatase-Konjugat (Promega, Bestell- Nr. P 3731).

Durch das Screening der genomischen lambda gtII-Genbank mit dem Antiserum gegen die 41kD Proteinbande konnten zwei sehr intensiv reagierende Klone (41-2 und 41-7) sowie dreizehn weitere schwächer reagierende Klone (41-1, 41-3, 41-4, 41-5, 41-6, 41-8, 41-9, 41-10, 41-12, 41-13, 41-14, 41-15 und 41-17) und etwa 40 weitere sehr schwach reagierende Klone identifiziert werden. Die Insert-DNA's der fünfzehn Klone, die 140bp bis 650bp betragen, wurden mit EcoRI herausgeschnitten und zur weiteren Charakterisierung in die EcoRI-Stelle des Vektors pUC8 kloniert.

Beispiel 2

Sequenzierung der Insert-Fragmente Klone 41-1 bis 41-10, 41-12 bis 41-15 und 41-17

Die Sequenzierung der Insert-DNAs erfolgte nach der Didesoxymethode mit Hilfe eines Primers und eines Reverse Primers direkt von den pUC8-Plasmiden (E.Y. Chen and P.H. Seeburg (1985), DNA 4, 165-170). Die Tabellen 1-15 zeigen die malaria-spezifischen DNA-Sequenzen und die daraus abgeleiteten Aminosäuresequenzen der Klone 41-1, 41-2, 41-3, 41-4, 41-5, 41-6, 41-7, 41-8, 41-9, 41-10, 41-12, 41-13, 41-14, 41-15 und 41-17 in den einzig möglichen offenen Leserahmen. Innerhalb dieser 15 Sequenzabschnitte findet man keinerlei Überlappungen und Homologien. Mit Hilfe des UWGCG (University of Wisconsin, Genetic Computer Group)-Progamms wurden diese 15 Sequenzen auf homologe Sequenzabschnitte innerhalb der EMBL-Datenbank untersucht. Keine dieser 15 Partialsequenzen oder größere homologe Abschnitte sind bisher beschrieben worden. Lediglich die Sequenz des Klons 41-10 besitzt von Nukleotid 1 bis 134 eine 74 %ige Homologie mit einer Teilsequenz von Nukleotid 2144 bis 2274 des 140kD Protein Gens, wie in der Anmeldung DE-P 37 41 057 vorgeschlagen. Die Sequenz des Klons 41-10 ist auch die einzige, welche für P.falciparum-Proteine typische repetitive Sequenzabschnitte beinhaltet. Die Aminosäuresequenz dieses Klons verfügt über drei Tetrapeptide der Sequenz Pro-Ser-Glu-Ser, wobei der zweite Serinrest des zweiten Repeats, verursacht durch eine G-A Transition, durch einen Asparagin-Rest ersetzt ist.

Weiterhin besitzt die Sequenz des Klons 41-7 von Nukleotid 50 bis 163 eine 56 %ige Homologie mit einer Aldolase mRNA von Nukleotid 218 bis 331 aus der Ratte (T. Mucai et al. (1986), J. Biol. Chem. 261, 3347-3354).

Beispiel 3

Nachweis des 41kD Antigens mit Hilfe von spezifischen Antikörpern gegen die Expressionsklone 41-2 und 41-7

Aus dem Antiserum gegen die 41kD Proteinbande wurden nach der Methode von L.S. Ozaki (1986, a.a.O.) Antikörper isoliert, die spezifisch gegen die Produkte der Expressionsklone 41-1, 41-2, 41-3, 41-7, 41-8 und lambda gtII (Kontrolle) gerichtet sind.

Zur Gewinnung von Schizonten wurde P.falciparum in Humanerythrozyten kultiviert (W. Trager und J.B. Jensen (1976), Science 193, 673-675) und durch Behandlung mit Sorbit synchronisiert (C. Lambros und J.P. Vanderberg (1979), J.Parasitol. 65, 418-420). Eine Anreicherung der Schizonten auf ca. 90 % wurde durch Flotation in Gelafundin[(R)] (Braun Melsungen) erzielt (analog G. Pasvol et al. (1978), Ann.Trop.Med.Parasitol. 72, 87-88). Die Schizonten wurden abzentrifugiert, gewaschen, 5 min. in SDS-Probenpuffer auf 100°C erhitzt, mit Ultraschall behandelt und aliquotiert eingefroren.

Aliquots der Schizontenlösung wurden für die Western blot Analyse der oben erwähnten spezifischen Antikörper verwendet (D.A. Johnson et al. (1984) Gene Anal. Tech. 1, 3-8). Dabei reagierten die Antikörper, die mit Hilfe der Expressionsklone 41-2 und 41-7 isoliert wurden, sehr intensiv mit einer 41kD Antigenbande aus Schizonten.

Beispiel 4:

Klonierung eines DNA-Fragmentes, das die genetische Information des Klons 41-2 beinhaltet

15 μg genomische DNA des P.falciparum-Stammes FCBR, die durch Lyse von Schizont-Kulturen mit anschließender Ethidiumbromid-CsCl-Zentrifugation gewonnen wurden (P. Oquendo et al. (1986) Molecular and Biochemical Parasitology 18, 89-101), wurde mit dem Restriktionsenzym EcoRI verdaut, nach der Vorschrift des Herstellers auf Gene Screen Membranen (Dupont) geblottet und anschließend mit nicktranslatierter Insert-DNA des Klons 41-2 mit einer spezifischen Aktivität von $10^7$ bis $10^8$ dpm/μg hybridisiert. Nach dem Waschen des Filters in 0,3xSSC (1xSSC: 0,15 M NaCl, 0,015 M NA-Citrat) und 1 % SDS (Natriumdodecylsulfat) bei 65°C für 1 h wurden die Filter autoradiographiert. Mit Hilfe dieses Southern Blot Experimentes wurde ein ca. 3kb großes genomisches EcoRI Fragment identifiziert, das mit der Insert-DNA des Klons 41-2 hybridisiert. In einem präparativen Gel wurden 60 μg mit dem Restriktionsenzym EcoRI geschnittene P.falciparum DNA des Stammes FCBR aufgetrennt, die Region von 2,8kb bis 3,2kb wurde ausgeschnitten und elektroeluiert (B. Perbal (1984), A Pratical Guide to Molecular Cloning). Diese DNA wurde nach der Methode von T.V. Huynh et al. (in DNA cloning Vol. I, ed. D.M. Glover (1985), 49-88) in den Vector lambda gt10 kloniert. $10^5$ PFU der erhaltenen Genbank wurden mit nicktranslatierter Insert-DNA des Klons 41-2 nach bekannten Methoden (T. Maniatis et al. (1982), Molecular Cloning, A Laboratory Manual) gescreent. Daraus resultierten mehrere Phagenklone, die mit der Insert-DNA des Klons 41-2 hybridisierten. Die Phagen DNA eines dieser Klone wurde isoliert (R.W. Davis et al. (1980), A Manual for Genetic Engineering, Advanced Bacterial Genetics), und mit dem Restriktionsenzym EcoRI verdaut; ein 3,0kb großes DNA-Fragment wurde gelelektrophoretisch gereinigt und in die EcoRI-Restriktionsstelle des Vektors pUC18 subkloniert (Plasmid pUC 41-2gen). In der anschließenden Southern-Blot Analyse (T. Maniatis et al., a.a.O.) hybridisierte dieses 3,0kb EcoRI-DNA-Fragment des pUC 41-2gen, mit der Insert-DNA des Klons 41-2.

Beispiel 5:

Sequenzanalyse des Klons pUC 41-2gen

Die Plasmid-DNA pUC 41-2gen wurde mit Hilfe eines Primers und Reverse-Primers von den EcoRI-Randstellen aus sequenziert (E.Y. Chen and P.H. Seeburg (1985) a. a. O.). Daraus konnten von den Enden des 3,0 kb EcoRI- Fragmentes jeweils etwa 250 Basen bestimmt werden. Die Sequenz eines dieser Enden ist dabei identisch mit der Insert-DNA des Klons 41-2. Zur Erstellung einer Restriktionskarte wurden je 0,5 μg des isolierten 3,0 kb EcoRI-DNA-Fragmentes mit unterschiedlichen Restriktionsenzymen inkubiert, gelelektrophoretisch aufgetrennt, auf Nitrocellulose geblottet und mit nicktranslatierter Insert-DNA des Klons 41-2 nach bekannten Methoden (T. Maniatis, a.a.O.) hybridisert. Anhand der Größe der zu hybridisierenden Restriktionsfragmente konnte auf die Entfernung verschiedener Restriktionsschnittstellen zur EcoRI-Schnittstelle, die den beiden Klonen 41-2 und pUC 41-2gen gemeinsam ist, geschlossen werden. Ausgehend von der so erstellten Restriktionskarte wurden Restriktionsfragmente des Klons pUC 41-2gen isoliert und zur Sequenzierung in die Phagenvektoren M13mp18 und M13mp19 subkloniert (F. Sanger et al. (1977), Proc.Natl.Acad.Sci. USA, 74, 5463-5467). Die Sequenz wurde dabei von der dem Gen eigenen EcoRI-Restriktionsstelle in Richtung zum 5′-Ende des Gens bis zu einer DraI-Restriktionsschnittstelle bestimmt. Die Tabelle 16 zeigt die DNA-Sequenz und die abgeleitete Aminosäuresequenz dieses 1230bp umfassenden DraI-EcoRI-DNA-Fragmentes des Klons pUC 41-2-gen. Die Sequenz von Position 1036 bis 1228 ist identisch mit der Insert-DNA von 41-2. Da die Sequenz des Klons 41-2 und die genomische Sequenz des Klons pUC 41-2gen von unterschiedlichen P.falciparum-Stämmen herrühren (Stamm T996 aus Thailand und Stamm FCBR aus Kolumbien), scheint zumindest dieser Genabschnitt sehr konserviert zu sein. Der offene Leserahmen dieser Sequenz beginnt in Position 784 mit einem ATG-Startcodon und endet mit einem TTC-Codon, welches zu der dem Gen internen EcoRI-Restriktionsschnittstelle gehört. Dieser Teil codiert für die 149 N-terminalen Aminosäuren des Proteins. Die Partialsequenz dieses Gens verfügt über keine repetitiven Sequenzanteile. Die abgeleitete Aminosäuresequenz beginnt mit einem Sequenzabschnitt von 18 Aminosäuren, von denen 4 sauer und 5 basisch sind. Gefolt wird dieser Sequenzabschnitt von einem hydrophoben Teil bestehend aus 11 Resten, der von beiden Seiten durch saure Aminosäurereste flankiert ist. Diese hydrophobe Region könnte als Signalsequenz fungieren. Die Region mit basischen und sauren Aminosäuren vor dieser putativen Signalsequenz ist relativ lang; ähnlich lange Regionen wurden jedoch auch für andere P.falciparum Proteine beschrieben (T. Triglia et al. (1987), The EMBO Journal 6, 1413-1419). Die abgeleitete Aminosäuresequenz wurde mit Hilfe des UWGCG-Programms auf hyrophile Regionen und Oberflächenbereiche sowie auf potentielle immunogene Epitop-Bereiche untersucht. Dabei wurden drei hydrophile Bereiche des Proteins ermittelt, die durch die Nukleotidsequenzen der Positionen 890 bis 907, 1079 bis 1093 und 1151 bis 1168 codiert werden.

Der 5′ nicht codierende Bereich des Gens ist extrem AT-reich (AT-Gehalt = 88,8 %), wie dies auch für andere P.falciparum Gene beschrieben wurde (J.L. Weber (1987), Gene 52, 103-109) und verfügt in jedem der drei Leserahmen über jeweils mehr als 15 Stopcodons. Weiterhin ist in Position 274 eine mögliche CAAT-Box vorhanden, von der 64 Nukleotide "down stream" eine mögliche TATA-Box lokalisiert ist. Diese Strukturen könnten eine mögliche Promotorregion für dieses Gen spezifizieren.

Beispiel 6:

Isolierung des vollständigen Gens für 41-2

Die Sequenzanalyse des Klons pUC 41-2gen ergab eine Sau3A Schnittstelle, die 947 bp von der EcoRI-Schnittstelle entfernt ist. Durch genomische Southern Blot Analyse wurde ein 2,0 kb großes Sau3A Fragment identifiziert, das mit der Insert-DNA des Klons 41-2 hybridisiert (vgl. Beispiel 4). Somit sollte dieses Sau3A Fragment in 3′ Richtung von der EcoRI-Stelle etwa über 1050 bp der genetischen Information des 41-2 Gens verfügen. In einem präparativen Gel wurden 60 $\mu$g mit dem Restriktionsenzym Sau3A geschnittene DNA des Stammes FBCR aufgetrennt und DNA-Fragmente von 1.8 kb bis 2.2 kb isoliert. Diese DNA wurde wie vom Hersteller (Stratagene) angegeben in die XhoI-Stelle des Vektors lambda ZAP kloniert. $10^5$ PFU dieser Genbank wurden mit nicktranslatierter Insert-DNA des Klons 41-2 gescreent und etwa 40 Phagenklone wurden identifiziert (vgl. Beispiel 4). Von einem dieser Phagenklone wurde durch automatische Excision nach der Methode von Stratagene eine Blueskript-Vektor (psK⁻ 41-2gen) isoliert. Durch Restriktion dieser Plasmid-DNA mit KpnI und EcoRI konnten zwei DNA-Fragmente von 950 bp und 1050 bp isoliert werden, von denen das 950 bp Fragment im Southern Blot mit der Insert-DNA des Klons 41-2 hybridisierte. Die Plasmid-DNA pSK⁻ 41-2 wurde mit Hilfe eines Primers und Reverse Primers sequenziert (E.Y. Chen und P.H. Seeburg (1985) a.a.O.). Die Sequenz, die mit Hilfe des Primers sequenziert wurde, ist identisch mit der Sequenz der Insert-DNA des Plasmids pUC 41-2gen von der Sau3A stelle in 3′ Richtung. Das 1050 bp EcoRI DNA Fragment des Plasmids pSK⁻ 41-2gen wurde in die EcoRI-Stelle des Vektors pKS⁺ subkloniert (T. Maniatis et al., a.a.O.). Von diesem DNA-Fragment wurde eine Restriktionskarte erstellt. Ausgehend von dieser wurden Restriktionsfragmente in die Bluescript-Vektoren subkloniert und nach der ssDNA Präparation sequenziert. (Instruction manual von Stratagene). Die Sequenz wurde dabei von der EcoRI-Restriktionsstelle des 41-2 Gens in 3′ Richtung bis zur Sau3A-Stelle vollständig bestimmt. Die Tabelle 17 zeigt in Fortsetzung zur Tabelle 16 die DNA-Sequenz und die abgeleitete Aminosäuresequenz dieses 1050 bp umfassenden EcoRI-Sau3A-DNA-Fragmentes des Klons pSK⁻ 41-2gen. Dieser Genabschnitt codiert lediglich noch für 35 zusätzliche Aminosäuren, bis ein TAG Stopcodon folgt. Der 3′ nicht codierende Bereich des Gens ist extrem AT-reich (AT-Gehalt = 84,7 %) und enthält in jedem der drei Leserahmen jeweils mehr als 11 Stopcodons. Die S1-Mapping-Technik (F.M. Ausubel et al. (1987), Current Protocols in Molekular Biology, Harvard Medical School, Boston) ergab keinerlei Hinweise für einen Intron-Exon-Aufbau dieses Gens. Schließlich wurde durch Northern Blot Analyse (T. Maniatis et al., a.a.O) eine mRNA des Schizontenstadiums in der Größe von 1.6 kb detektiert. Daher muß angenommen werden, daß das 41-2 Gen nur einen einzigen codierenden Abschnitt von 552 bp besitzt (AT-Gehalt = 73 %). Dieser codiert für ein Antigen von 21512 Dalton, welches eine Signalsequenz (vgl. Beispiel 5), aber keine repetitiven Abschnitte besitzt. Dieses Antigen verfügt neben der Signalsequenz über zwei weitere hydrophobe Abschnitte in den Aminosäurepositionen 73 bis 85 und 130 bis 147, die eventuell eine Funktion für die Membranverankerung besitzen.

Beispiel 7:

Expression der Inserts der Klone 41-1 bis 41-5 sowie 41-7, 41-10 und 41-14 in dem Vektor pEX31

Die Insertfragmente der Klone 41-1 bis 41-5, 41-7, 41-10 und 41-14 wurden nach Restriktion mit EcoRI gelelektrophoretisch isoliert, in den dephosphorylierten, mit dem Restriktionsenzym EcoRI verdauten Vektor pEX31b ligiert (K. Strebel et al. (1986) Journal of Virology 57, 983-991) und in kompetente, das Plasmid pCI857 (F. Remaut et al. (1981), Gene 15, 81-93) enthaltende C600-Bakterien transformiert. Einzelne Kolonien wurden mittels SDS-PAGE auf Expression der Plasmodien-spezifischen DNA-Sequenzen als MS2-Polymerase-Fusionsproteine hin untersucht. Die Induktion erfolgte durch Temperaturerhöhung nach der Methode von H. Küpper et al. (in Y.Ikeda and T. Beppu (ed). Proceedings of the Fourth International Symposium on Genetics of Industrial Mikroorganisms (1982), Kyoto Kodansha Ltd., Tokyo). Alle 8 Fragmente konnten in hoher Ausbeute zur Expression gebracht werden.

Beispiel 8:

Reinigung der Expressionsprodukte

Kulturen von transformierten Bakterien wurden jeweils in 1 l LB-Medium mit 50 µg/ml Ampicillin und 25 µg/ml Kanamycin bei 28°C 20 h heftig geschüttelt. Nach Zugabe von 4 l auf 42°C erwärmtem LB-Medium wurde erneut 4 h bei 42°C geschüttelt. Die Bakterien wurden abzentrifugiert, in 200 ml phosphatgepufferter Kochsalzlösung (PBS) resuspendiert und mechanisch aufgeschlossen. Die löslichen Proteine wurden durch Zentrifugation abgetrennt und die Sedimente, die die Expressionsprodukte enthielten, nach zweimaligem Waschen mit PBS nacheinander mit steigenden Harnstoffkonzentrationen (von 1 M bis etwa 5 M) gewaschen, bis die Fusionsproteine in Lösung gingen. Anschließend wurde mit fallenden Harnstoffkonzentrationen bis zu der Harnstoffkonzentration dialysiert, die ausreichte, um die Expressionsprodukte in Lösung zu halten. Dieses Verfahren führte zu einem Reinheitsgrad von 60-80 %.

Beispiel 9:

Nachweis des Antigens, das durch 41-2gen codiert wird

Mittels Kaninchen-Antiseren, die gegen das Expressionsprodukt des Klons 41-2 gerichtet sind, ließ sich im Western Blot mit Schizontproteinen kein Malariaantigen eindeutig nachweisen. Deshalb sollte ein größeres DNA-Fragment des Gens 41-2 exprimiert werden. Dazu wurde mit Hilfe der Restriktionsenzyme AluI und EcoRI ein 383 bp Fragment des 41-2 Gens isoliert, daß 70 % der codierenden Region beinhaltet. Dies wurde in die SmaI-und EcoRI-Stellen des Plasmids pUC18 subkloniert. Aus diesem Plasmid wurde mit Hilfe des Restriktionsenzyms EcoRI ein 401 bp Fragment isoliert und in die EcoRI-Stelle des Vektors pEX31b kloniert. Nach der Transformation konnten Bakterienkolonien identifiziert werden, welche ein Fusionsprotein von 26 kD exprimieren (vgl. Beispiel 7). Dieses wurde gereinigt (vgl. Beispiel 8) und zur Immunisierung von Kaninchen eingesetzt. Die Antiseren erkennen im Western Blot mit Schizontenproteinen ein 29 kD Antigen, das durch das 41-2 Gen codiert wird. Die Differenz von dem kalkulierten Molekulargewicht von 22 kD zu dem Molekulargewicht von 29kD in SDS-Polyacrylamidgelen kann durch Sekundärmodifikation erklärt werden. So besitzt das Protein in dem Asparagin-Rest in Position 166 eine N-Glykosylierungsstelle.

Daß von dem Klon 41-2 ein recht kleines antigen codiert wird, wird auch durch Northern Blot Analyse bestätigt. So wurde im Schizontenstadium von P.falciparum eine mRNA von 1,6 kb detektiert, welche mit der Insert-DNA des Klons 41-2 hybridisiert. Die Northern Blot Analyse wurde nach bekannten Methoden (T. Maniatis et al., a.a.O.) durchgeführt.

Beispiel 10:

Antigenzuordnung weiterer Klone

Antiseren gegen die gereinigten Fusionsproteine der Klone 41-1, 41-3, 41-4, 41-5, 41-7 und 41-10 wurden dazu benutzt, die korrespondierenden Antigene durch Western Blot Analyse mit Schizontproteinen zu identifizieren. Dabei ließen die Antiseren gegen die Fusionsproteine der Klone 41-1, 41-3 und 41-4 keine eindeutigen Identifizierungen zu. Die Insert-DNA des Klons 41-5 kann einem 96 kD Antigen zugeordnet werden. Eine Dreiergruppe von 96 kD Antigenen von P.falciparum wurde beschrieben (H. Jouin et al. (1987), Inf. Imm. 55, 1387-1392). Antiseren, die gegen ein Expressionsprodukt des Klons 41-10 gerichtet sind, erkennen zwei Antigene von 113 und 140 kD. Die Reaktion mit dem 113 kD-Antigen wurde als Kreuzreaktion mit dem SERPI Antigen (vgl. Beispiel 2) identifiziert, das mit einem protektiven Antigen identisch ist. 41-10 codiert somit eine 140 kD Protein von P.falciparum.

Gemeinsam ist all diesen Genen, daß die von ihnen codierten Antigene oder Teile davon mit einem Serum reagieren, das gegen eine protektiv wirkende 41 kD Proteinbande gerichtet ist. Nur der Klon 41-7, der neben dem Klon 41-2 die stärkste Reaktivität mit dem Antiserum gegen die 41 kD Proteinbande besitzt, kann eindeutig einem 41 kD Protein zugeordnet werden.

Antiseren, die gegen das Fusionsprotein des Klons 41-7 gerichtet sind, erkennen im Western Blot mit Schizontproteinen eindeutig die 41 kD Proteinbande, die auch von dem Ausgangsserum erkannt wird. Dieser Klon scheint somit ein Teilfragment des 41 kD Antigens zu codieren. Daß die untersuchten Klone Teilsequenzen verschiedener Gene tragen, wurde für die Klone 41-1, 41-2, 41-3, 41-7, 41-10, 41-14 und 41-15 auch durch Southern Blot Analysen bestätigt.

Beispiel 11:

Herstellung einer genomischen lambda gtll Genbank

2 $\mu$g DNA des P.falciparum Stammes FCBR wurden über Nacht bei 37 °C mit 14 Einheiten des Restriktionsenzyms EcoRI in 10 mM Tris-HCl (pH 7,5), 10 mM MgCl$_2$, 1 mM Dithiothreitol und 40 % Glycerin inkubiert und gelelektrophoretisch aufgetrennt. Unter diesen Bedingungen zeigt das Restriktionsenzym EcoRI Stern-Aktivität, wodurch sich DNA-Fragmente von etwa 50 bp bis 10 kb bilden. Die DNA-Region zwischen 500 bp und 7 kb wurde elektroeluiert und nach der Methode von T.V. Huynh et al. (1985; in DNA cloning Vol. I, a practical approach, ed. D.M. Glover) in den Vektor lambda gtll kloniert. Es wurde eine Genbank von 5 x 10$^5$ rekombinanten Phagenklonen hergestellt, welche amplifiziert wurde.

Beispiel 12:

Schutzversuch im Tiermodell: Immunisierung von Aotus lemurinus griseimembra (Karyotyp VI)

Dieses Experiment wurde durchgeführt, um die Wirksamkeit der beschriebenen Expressionsprodukte bezüglich der Induktion von schützender Immunität in für P. falciparum empfänglichen Affen zu prüfen. Als Versuchsimpfstoff diente eine Kombination der Expressionsprodukte der immunologisch stark reagierenden Klone 41-1, 41-2 und 41-3.

1. Versuchsanordnung

6 Aotusaffen der o.g. Spezies (1000-1500 g Körpergewicht, männliche und weibliche Tiere aus der Zucht der Behringwerke AG) wurden randomisiert und in 2 Gruppen zu je 3 Tieren aufgeteilt.

Fusionsproteine der Klone 41-1, 41-2 und 41-3 wurden in PBS 3 M Harnstoff gelöst und im Verhältnis 1:1:1 gemischt. (Endkonzentration: 300 $\mu$g Protein/ml). 3 Tiere wurden in Abständen von 3 Wochen 3 x mit jeweils 1 ml der kombinierten Fusionsproteine subkutan immunisiert. Als Adjuvans diente eine 10%ige Zumischung von 50% Al(OH)$_3$/ 45% Lecithin/5% Saponin zum Antigen.

3 Tiere der Infektions-Kontrollgruppe erhielten ebenfalls nach o.g. Schema jeweils eine Injection aus PBS 3 M Harnstoff + Adjuvans ohne Antigenkomponente.

Um eine möglichst gleichstarke experimentelle P.falciparum-Infektion in den Tieren zu gewährleisten, wurden alle Affen acht Tage nach der letzten Immunisierung splenektomiert (erhöhte Suszeptibilität).

Am 67. Tag nach der 1. Vaccinierung wurden alle 6 Tiere mit 5 x 10$^6$ parasitierten Erythrozyten intravenös infiziert. Als Challenge-Stamm wurde P. falciparum-Palo Alto (Uganda) gewählt, der, in vitro auf Aotus-Erythrozyten adaptiert, von einem splenektomierten Spendertier (4 % Parasitämie) direkt auf die Versuchstiere übertragen wurde. Dieser Stamm zeichnet sich durch eine hohe Infektiosität im Vergleich zu anderen P. falciparum-Isolaten aus. Es ist außerdem interessant zu erwähnen, daß dieser Stamm heterolog zu dem für die Gewinnung der Immunisierungsantigene verwendeten Stamm T996 (Thailand) bezüglich Provenienz und Serotyp ist.

Während der gesamten Verlaufsstudie (vor und nach Immunisierung sowie nach Challenge) wurden physiologische, parasitologische, serologische und klinischchemische Parameter regelmäßig untersucht.

2. Ergebnisse

Während der gesamten Immunisierungsphase zeigten sich keine pathologischen Veränderungen aller untersuchten physiologischen (klinisches Erscheinungsbild, Temperatur, Gewicht) und klinisch- chemischen Parameter (Erythrozyten, Hämatokrit, Blutsenkung, Serumenzyme GPT und GOT). Zusätzliche sicherheitspharmakologische Untersuchungen (akute subkutane Toxizität in der Maus, lokale Verträg- lichkeit am Affen nach Richtlinien der Europäischen Pharmakopoe) bescheinigten der verwendeten Impfstoff-Präparation ausreichende Sicherheit und Verträglichkeit.

2.1 Parasitämie

Hauptparameter für die Wertbemessung eines induzierten Schutzes (Protektion) ist der mikroskopische Nachweis parasitierter Erythrozyten im peripheren Blut des Versuchstieres.

Bereits vom 7.-10. Tag nach der Infektion konnten vereinzelt parasitierte Erythrozyten (kleiner als 1 Promille) im Giemsa-gefärbten Blutausstrich der nicht immunisierten Tiere nachgewiesen werden. Die immunisierten Tiere zeigten ein verzögertes Auftreten von Parasiten vom 10.-15. Tag p.i., erreichten maximale Parasitämien von 1-2 % und kontrollierten die Infektion spontan. Ein Tier starb interkurrent an Pneumonie.

Während ein Tier der nicht immunisierten Gruppe eine maximale Parasitämie von 4,5 % selbst kontrollieren konnte, mußten die beiden anderen Tiere nach Erreichen einer Parasitämie von > 10 % mit Mefloquin [R] (Hoffmann La Roche) behandelt werden, um einen tödlichen Infektionsverlauf zu verhindern. Der Challenge-Stamm Palo Alto erwies sich in vorausgegangenen Infektionsversuchen als Chloroquin-resistent.

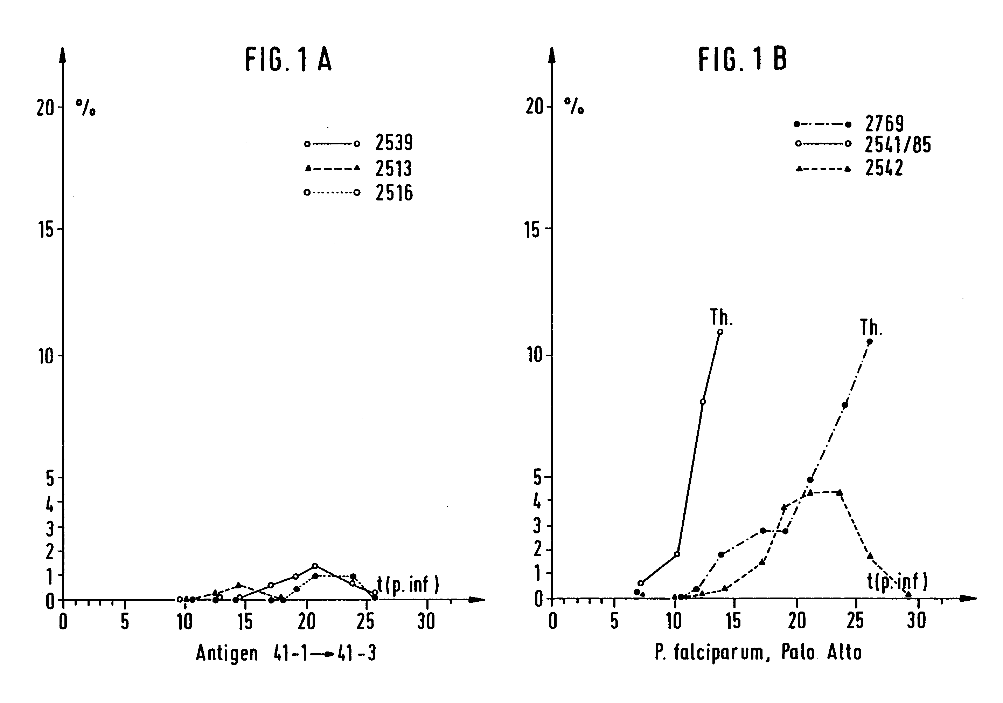

Figur 1 zeigt links den Verlauf der Parasitämie in Aotus-Affen nach Immunisierung mit einer Kombinationsvaccine bestehend aus Fusionsproteinen der Klone 41-1, 41-2 und 41-3, rechts den der Kontrolle (nicht immunisierte Tiere).

## TABELLE 1:

### Nukleotidsequenz des malaria-spezifischen DNA-Inserts des Klons 41-1 und abgeleitete Aminosäuresequenz

```
           10              30              50
TAAAATCTTTATGTATTTTTAAACAAAGAAATAAAAAGGTAAGGTCATCAAATAATTCAT
  LysSerLeuCysIlePheLysGlnArgAsnLysLysValArgSerSerAsnAsnSerS

           70              90              110
CTTTCTTAATTGATTTTAGAAACTCACATACGAATAATATCAATATGTTAACAGAAAATC
  erPheLeuIleAspPheArgAsnSerHisThrAsnAsnIleAsnMetLeuThrGluAsnG

          130             150             170
AAAAGTTTAATAATGTATTATTGAATAAAGAAATTCAGATGGATGAAAATCAGGAACGTG
  lnLysPheAsnAsnValLeuLeuAsnLysGluIleGlnMetAspGluAsnGlnGluArgG

          190             210             230
AATTTTCAATTGATGATTGTTTAATGAACTGCCTGAAACATAATGCATCTAATTTAAAAA
  luPheSerIleAspAspCysLeuMetAsnCysLeuLysHisAsnAlaSerAsnLeuLysT

          250             270             290
CCAATGAAGATTATGAAAGATATTGTAATGAAGACAAAATATTGAATCCAGATTATAAGC
  hrAsnGluAspTyrGluArgTyrCysAsnGluAspLysIleLeuAsnProAspTyrLysP

          310             330             350
CTTCAGAATCTAGAAAACTGGGAGAAAAATTTTTGCAAAAAAGTCAAAAGGAATTATATT
  roSerGluSerArgLysLeuGlyGluLysPheLeuGlnLysSerGlnLysGluLeuTyrT

          370
ATTCTTATGC
  yrSerTyr
```

9

TABELLE 2:

Nukleotidsequenz des malaria-spezifischen DNA-Inserts des Klons 41-2 und abgeleitete Aminosäuresequenz

```
          10              30              50
AACATGTGTGGAAATATTTATTTCAGCATTCATCTGATTTACTTAAATCTCAAGATAGTA
 HisValTrpLysTyrLeuPheGlnHisSerSerAspLeuLeuLysSerGlnAspSerI

          70              90             110
TTTATGAGTATATGATATGTGATAAAAATATTTTATTAAATAAATTTATAAATGTACCAA
 leTyrGluTyrMetIleCysAspLysAsnIleLeuLeuAsnLysPheIleAsnValProL

         130             150             170
AAGATTATGGAAATATAAATTGTGCTGCCTTTGCAGCAGGTATTGTTGAAGGCTTCCTCT
 ysAspTyrGlyAsnIleAsnCysAlaAlaPheAlaAlaGlyIleValGluGlyPheLeuC

         190
GTAGTTCTGAATTC
 ysSerSerGluPhe
```

TABELLE 3:

Nukleotidsequenz des malaria-spezifischen DNA-Inserts des Klons 41-3 und abgeleitete Aminosäuresequenz

```
          10              30              50
CAGTAAAAATTTTAAAAAAAAAGAAAAATTTAAGAAAAATAAAGGAAACCACTGATGAAG
  ValLysIleLeuLysLysLysLysAsnLeuArgLysIleLysGluThrThrAspGluG

          70              90             110
AGAAAACTTCAGATAATGTTTCTCAAATGTATGAAAGAAAAGGTGGACCATTACCACCAC
 luLysThrSerAspAsnValSerGlnMetTyrGluArgLysGlyGlyProLeuProProP

         130
CCGAACTTAGAAAACA
 roGluLeuArgLys
```

TABELLE 4:

Nukleotidsequenz des malaria-spezifischen DNA-Inserts des Klons 41-4 und abgeleitete Aminosäuresequenz

```
          10              30              50
GTATACCTGAATTTTTAGGACAATATCATAATGTTCCCCATGTATTCAAAGATTATATGA
 IleProGluPheLeuGlyGlnTyrHisAsnValProHisValPheLysAspTyrMetS

          70              90             110
GTTCCAATGATTTTATAAGTGGTATAAATAATATAAATGAATCAGATGCTCTTTTTAATA
 erSerAsnAspPheIleSerGlyIleAsnAsnIleAsnGluSerAspAlaLeuPheAsnA

         130             150
ACATACAATATATAAACCAAGCGAATGACCAAGAAGAAAACAAATT
 snIleGlnTyrIleAsnGlnAlaAsnAspGlnGluGluAsnLys
```

## TABELLE 5:

Nukleotidsequenz des malaria-spezifischen DNA-Inserts des
Klons 41-5 und abgeleitete Aminosäuresequenzen

```
            10                    30                    50
TGTTTGATAATAGTGATTTTATTAAATCAATAATGGATTCTAATAAACAATTAAAAAAGT
  PheAspAsnSerAspPheIleLysSerIleMetAspSerAsnLysGlnLeuLysLysL

            70                    90                   110
TAAGAGAACAAAATTCTGATTTAAATCATATTTTAAATGATTCTCAGACTTTAAAACAAT
euArgGluGlnAsnSerAspLeuAsnHisIleLeuAsnAspSerGlnThrLeuLysGlnS

           130                   150                   170
CTTTTGAAATGATTAAGAATCCATCTTTGATGAAAGAATTAATGAAAAATACTGATAGAG
erPheGluMetIleLysAsnProSerLeuMetLysGluLeuMetLysAsnThrAspArgA

           190
CTATTAGTAATATTGAAGCCATACC
laIleSerAsnIleGluAlaIle
```

## TABELLE 6:

Nukleotidsequenz des malaria-spezifischen DNA-Inserts des
Klons 41-6 und abgeleitete Aminosäuresequenz

```
            10                    30                    50
TCGTTGTCCTTTCCTTTGGTGATAAACGCAATGAGATAAAACAAAAAATTGACACGTTTT
  ValValLeuSerPheGlyAspLysArgAsnGluIleLysGlnLysIleAspThrPheC

            70                    90                   110
GTGGTGTAAGTAATGAAGAAAAGGAGAAACTAAAGGAACAATGGAAATGCTATGAAGCTA
ysGlyValSerAsnGluGluLysGluLysLeuLysGluGlnTrpLysCysTyrGluAlaL

           130                   150
AATATGTAAAGGAAGATAATAAAAGTAAAG
ysTyrValLysGluAspAsnLysSerLys
```

TABELLE 7:
Nukleotidsequenz des malaria-spezifischen DNA-Inserts des
Klons 41-7 und abgeleitete Aminosäuresequenz

```
            10                30                50
TGAATGCCCCAAAAAAATTACCAGCAGATGTTGCCGAAGAATTAGCAACCACCGCCCAAA
  AsnAlaProLysLysLeuProAlaAspValAlaGluGluLeuAlaThrThrAlaGlnL

            70                90                110
AGCTTGTTCAAGCTGGAAAGGGAATTTTAGCTGCTGATGAATCAACACAAACCATTAAGA
ysLeuValGlnAlaGlyLysGlyIleLeuAlaAlaAspGluSerThrGlnThrIleLysL

            130               150               170
AAAGATTCGACAACATCAAATTAGAGAACACAATAGAAAACAGAGCTAGCTACAGAGATT
ysArgPheAspAsnIleLysLeuGluAsnThrIleGluAsnArgAlaSerTyrArgAspL

            190               210               230
TATTATTTGGAACTAAAGGATTAGGAAAATTCATTTCAGGAGCAATTTTATTTGAAGAAA
euLeuPheGlyThrLysGlyLeuGlyLysPheIleSerGlyAlaIleLeuPheGluGluT

            250
CATTATTTCAAAAGAATGAAGCCGGT
hrLeuPheGlnLysAsnGluAlaGly
```

TABELLE 8:

Nukleotidsequenz des malaria-spezifischen DNA-Inserts des
Klons 41-8 und abgeleitete Aminosäuresequenz

```
            10                30                50
TAACATTTTCTGTAGATACAAAACAAAATTTAAATGCATCTTATTCTAGTGGACAAGAAA
  ThrPheSerValAspThrLysGlnAsnLeuAsnAlaSerTyrSerSerGlyGlnGluA

            70                90                110
ATAAACAAAATGAATCTGATGGAAAAGAAAATGAAGAAGATGAAGAAAATAAGGTATATG
snLysGlnAsnGluSerAspGlyLysGluAsnGluGluAspGluGluAsnLysValTyrA

            130               150               170
ATTTAATACTGGAAAATATAGAACCTAACAAAAAAATACCCATCATAAAAATCGTTAAAG
spLeuIleLeuGluAsnIleGluProAsnLysLysIleProIleIleLysIleValLysG

            190               210               230
AAATAAAAAAAGATCTTAATTTAAAACAAGCAAAGGATTTAGTTGATAATTTGCCACA
luIleLysLysAspLeuAsnLeuLysGlnAlaLysAspLeuValAspAsnLeuPro
```

TABELLE 9:

Nukleotidsequenz und abgeleitete Aminosäuresequenz des
malaria-spezifischen Inserts-DNA des KLons 41-9

```
        10                30                50
AAAATAAAAATTATACAGGTAATTCTCCAAGTGAAAATAATAAGAAAGTTAACGAAGCTT
  AsnLysAsnTyrThrGlyAsnSerProSerGluAsnAsnLysLysValAsnGluAlaL

        70                90                110
TAAAATCTTACGAAAATTTTCTCCCAGAAGCAAAAGTTACAACAGTTGTAACTCCACCTC
  euLysSerTyrGluAsnPheLeuProGluAlaLysValThrThrValValThrProProG

        130               150               170
AACCAGATGTAACTCCATCTCCATTATCTGTAAGGGTAAGTGGTAGTTCAGGATCCACAA
  lnProAspValThrProSerProLeuSerValArgValSerGlySerSerGlySerThrL

        190               210               230
AAGAAGAAACACAAATACCAACTTCAGGCTCTTTATTAACAGAATTACAACAAGTAGTAC
  ysGluGluThrGlnIleProThrSerGlySerLeuLeuThrGluLeuGlnGlnValValG

        250               270               290
AATCACAAAATTATGACGAAGAAGATGATTCCTTAGTTGTATTACCCATTTTTGGAGAAT
  lnSerGlnAsnTyrAspGluGluAspAspSerLeuValValLeuProIlePheGlyGluS

        310               330               350
CCGAAGATAATGACGAATATTTAGATCAAGTAGTAACTGGAGAAGCAATATCTGTCACAA
  erGluAspAsnAspGluTyrLeuAspGlnValValThrGlyGluAlaIleSerValThrM

        370               390
TGGATAATATCCTCTCAGGATTTGAAAATGAATATGA
  etAspAsnIleLeuSerGlyPheGluAsnGluTyr
```

TABELLE 10:

Nukleotidsequenz und abgeleitete Aminosäuresequenz des
malaria-spezifischen Inserts-DNA des Klons 41-10

```
        10                30                50
AATCTCATTCTGACGAAAATATTGTAACTTTACAAGGAAAACTTAGAAATACAGCTATCT
  SerHisSerAspGluAsnIleValThrLeuGlnGlyLysLeuArgAsnThrAlaIleC

        70                90                110
GTATAAAGAATGTTGATGAATGGATATTAAATAAAAGAGGTCTAACATTACCTAGTGAAT
  ysIleLysAsnValAspGluTrpIleLeuAsnLysArgGlyLeuThrLeuProSerGluS

        130               150
CACCTAATGAATCACCTAGTGAATCAGATAGTTATCTTAA
  erProAsnGluSerProSerGluSerAspSerTyrLeu
```

13

TABELLE 11:

Nukleotidsequenz des malaria-spezifischen DNA-Inserts des
Klons 41-12 und abgeleitete Aminosäuresequenz

```
        10                  30                  50
ATGAAGGAGAAGAATTAATATTAAATGATGATCAAAATAAATTACATATTGATACATTTG
  GluGlyGluGluLeuIleLeuAsnAspAspGlnAsnLysLeuHisIleAspThrPheG

       70                  90                  110
AAAAATACAAATATCTCATTTGTGAAAATATTAATAATGACAAATTTGTTATAAAAAATA
luLysTyrLysTyrLeuIleCysGluAsnIleAsnAsnAspLysPheValIleLysAsnA

      130                 150                 170
ATCAAATTACAACATTTGAAAACTTTTTGAAAAACCAACAAAATTTTGAAATAA
snGlnIleThrThrPheGluAsnPheLeuLysAsnGlnGlnAsnPheGluIle
```

TABELLE 12:

Nukleotidsequenz und abgeleitete Aminosäuresequenz des
malaria-spezifschen Inserts des Klons 41-13

```
              20                    40                    60
TAAATAATGAAAAATATGGATAAACAAAATGTTAATATTCAAAATGAAGGTAATGGTTTTA
   AsnAsnGluAsnMetAspLysGlnAsnValAsnIleGlnAsnGluGlyAsnGlyPheA

              80                   100                   120
ATAATAATAAAAATAATAATGATCTTTTAAATGTTTATATATCACCTAATATGATTAATC
 snAsnAsnLysAsnAsnAsnAspLeuLeuAsnValTyrIleSerProAsnMetIleAsnH

             140                   160                   180
ATTCTTTATCTTCAACTTGTGAAAAAAAAAATAAAGAAGATAACAAAATGAATGACAATA
 isSerLeuSerSerThrCysGluLysLysAsnLysGluAspAsnLysMetAsnAspAsnL

             200                   220                   240
AATTTCTTAATAGCAGTAGTAAAATGAAAATTCCAGAGATAAGTACGAACAACTCAAATG
 ysPheLeuAsnSerSerSerLysMetLysIleProGluIleSerThrAsnAsnSerAsnG

             260                   280                   300
AAAAGATTGTTAATGTGTCAAATGATGAAATGTTAGTATATCATAATTTAACCGTATTAA
 luLysIleValAsnValSerAsnAspGluMetLeuValTyrHisAsnLeuThrValLeuA

             320                   340                   360
ATGTAAAGGAACAAGGAGGTGTAACAGAAGAATCGAGCTGTATAAAACGCACATATTTTG
 snValLysGluGlnGlyGlyValThrGluGluSerSerCysIleLysArgThrTyrPheV

             380                   400                   420
TGGATCAATTTTATGATTCATATAATATGAGAAATGAAAAAATAACAGATGATAATATGC
 alAspGlnPheTyrAspSerTyrAsnMetArgAsnGluLysIleThrAspAspAsnMetG

             440                   460                   480
AAGTAGAAGATATATATAATGTAAAGGAAAATATAAAAAGAACTCTAAAAGGTGATGGTC
 lnValGluAspIleTyrAsnValLysGluAsnIleLysArgThrLeuLysGlyAspGlyP

             500                   520                   540
CTGATGATGTCAAAACGAATATGCTGAGTGAAGATAATAGTTATGCAAGTGGTTTATGGG
 roAspAspValLysThrAsnMetLeuSerGluAspAsnSerTyrAlaSerGlyLeuTrpG

             560                   580                   600
GTAACGAAATAAACTTTATTAGTAATAATGAAAATTGTTTAAATAGCTATGATATATCAT
 lyAsnGluIleAsnPheIleSerAsnAsnGluAsnCysLeuAsnSerTyrAspIleSerC

             620                   640
GTGATGAGAAATATATCCCAAATGAAGAGGAACAGGA
 ysAspGluLysTyrIleProAsnGluGluGluGln
```

TABELLE 13:

Nukleotidsequenz und abgeleitete Aminosäuresequenz des malaria-spezifischen Inserts des Klons 41-14


```
         10                30                50
ACAACAATATGAACAGAATAAATAGTTTAAACAATAAAAATAATATTAACCCTATAAATC
  AsnAsnMetAsnArgIleAsnSerLeuAsnAsnLysAsnAsnIleAsnProIleAsnG

        .70                90               110
AATACAATGATGAAAAACAAAACTTACTTAACGNCCATCTTCAGTYCAATCAAGTAAATT
 lnTyrAsnAspGluLysGlnAsnLeuLeuAsnXxxHisLeuGlnXxxAsnGlnValAsnT

        130               150               170
ATNATAATAACCTTGTGAATGGCYTTCATANAANNAAATTTTTAAGCAATAATAATTATA
 yrXxxAsnAsnLeuValAsnGlyXxxHisXxxXxxLysPheLeuSerAsnAsnAsnTyrI

        190               210               230
TTAATACTACAGATATTAATGGAAATAATATGATTAGTCATAATGATCATATGAATAATA
 leAsnThrThrAspIleAsnGlyAsnAsnMetIleSerHisAsnAspHisMetAsnAsnL

        250               270               290
AATTATACAGTAATATAAACAATAATTATTATTATAATAGGGCTAACAATGAAATTCCTA
 ysLeuTyrSerAsnIleAsnAsnAsnTyrTyrTyrAsnArgAlaAsnAsnGluIleProA

        310               330               350
ATAATAATAGTAACAATCATAATAATAATTTCAATATATATGAATCCAAATACCAAACCA
 snAsnAsnSerAsnAsnHisAsnAsnAsnPheAsnIleTyrGluSerLysTyrGlnThrM

        370               390               410
TGATTCATAACAACAACATAGGACAAGATCTAAAACAACAAATAAATAATTATAATGAAA
 etIleHisAsnAsnAsnIleGlyGlnAspLeuLysGlnGlnIleAsnAsnTyrAsnGluA

        430               450               470
ATACATCTTCTAATAATAATTTAAGTATATCTCAATTACTTGAGGGAAATACAAATTTTA
 snThrSerSerAsnAsnAsnLeuSerIleSerGlnLeuLeuGluGlyAsnThrAsnPheI

        490               510               530
TAAATATTTCTAATACATTTATTAATACGAATTATTCTAATGATTTTCATCA
 leAsnIleSerAsnThrPheIleAsnThrAsnTyrSerAsnAspPheHis
```

16

TABELLE 14:

Nukleotidsequenz und abgeleitete Aminosäuresequenz des
malaria-spezifischen Inserts des Klons 41-15.

```
          10                  30                  50
ACAGCAACAACAATAATAATAATAATAATAATATTAGTAATAATATTAGTAATAATAAAG
 SerAsnAsnAsnAsnAsnAsnAsnAsnAsnIleSerAsnAsnIleSerAsnAsnLysA

          70                  90                 110
ATTGTGATGAATACGATTATCAGGAAGAATATTTGAAAGATAAAGCATTATACGATTCAG
 spCysAspGluTyrAspTyrGlnGluGluTyrLeuLysAspLysAlaLeuTyrAspSerA

         130                 150                 170
ATATGGACGAAAATACAAATCAACTTCACAATAATGAACATCATACAAATCAACATCACG
 spMetAspGluAsnThrAsnGlnLeuHisAsnAsnGluHisHisThrAsnGlnHisHisA

         190                 210                 230
CAAATTGGCATCATCACAAACATCAAAAGCAACATTTCAAACAACTTATTGATCATAACA
 laAsnTrpHisHisHisLysHisGlnLysGlnHisPheLysGlnLeuIleAspHisAsnA

         250                 270
ATATGATAAATAATAATGATAATAATATTATCAATAA
 snMetIleAsnAsnAsnAspAsnAsnIleIleAsn
```

TABELLE 15:

Nukleotidsequenz und abgeleitete Aminosäuresequenz des
malaria-spezifischen..Inserts des Klons 41-17

```
          20                  40                  60
GCACTGCCACCCTTGTTGCGGAAGAATTGCACCAGCTCGGCTATTCACTGGCGCTGGGTC
 ThrAlaThrLeuValAlaGluGluLeuHisGlnLeuGlyTyrSerLeuAlaLeuGlyA

          80                 100
GCGAAGTAGTTAATGAAAGTAGCCGGATGGGATTACCTGATGAATTC
 rgGluValValAsnGluSerSerArgMetGlyLeuProAspGluPhe
```

TABELLE 16:

Nukleotidsequenz und abgeleitete Aminosäuresequenz des 5'Endes des P.falciparum (Isolat FCBR) Genes, das durch das    Insert des Klones 41-2 spezifiziert wird.

```
          10                  30                  50
TTTAAAAATTTTATAAATAATTTTTCTCTTTTATATTTAATACATCTATAAGTATATATG

          70                  90                 110
TAATAATTTGATACACAAGAAATGTGTATTTTTAATATATATATATATATATATATATAT

         130                 150                 170
ATATATATATATATATATATATATATATATGATATATATAAATATATACATTTATTTA

         190                 210                 230
TTCCATAATTTATTAAAAATAAATTTATATATTTTATTTTATTTTTTATTTATTTATTTG

         250                 270                 290
TATATATTAAATCTTTTCAATGGAATAAAATTCAATCGGATCGTTATATAAACTTTATTA

         310                 330                 350
TATCAAATAAAACACTTTTTATAATAATACGAAAAATATATTTCCTTATTTTTATGTTTT

         370                 390                 410
CAAAATTTTAGTAGACTTATAATATTATTATGGATAACATTAACAAATAAAAATATTATG

         430                 450                 470
AGTATAATATGTAAATTATTTTTTTTTTTTTACAGTTTATATGTTTATGAACATATAATG

         490                 510                 530
TGATAAATAAAATTGATTAATTATTATTATATATAATTACTCTTGTAATTTATTAAAATG

         550                 570                 590
GTATATTATATATATATATAATTTTTTTTATATTATTTGAATAAAAATATTAAATAAA

         610                 630                 650
AATTTTGTGTTTGGGTAAATCATAATAAGTGCTAACGTTCATAATTTATCTCATTAAAAA

         670                 690                 710
ATAGAAATGAAATATAATATTTACGACAGTACATATATATATGTATATTATTAAAAAA

         730                 750                 770
AAATAAAAATAACACATATATATATATATATATATATATATTGATAATATATGTTTTA

         790                 810                 830
AGTATGGATAAATCAAAAAGTTCCATAGAGAAAGAATTAAATAGGATAAAACAGGATGTG
   MetAspLysSerLysSerSerIleGluLysGluLeuAsnArgIleLysGlnAspVal

         850                 870                 890
AGCTTAAGCGCATTTAGTATCCTCTTTAGTGAAATGGTACAATATTGTTTATATAAAAGT
SerLeuSerAlaPheSerIleLeuPheSerGluMetValGlnTyrCysLeuTyrLysSer

         910                 930                 950
AAAAGAGGATATCGAATAGAAGATTGTTTACATGAAATGGGTTTACGTGTAGGTTATAAA
LysArgGlyTyrArgIleGluAspCysLeuHisGluMetGlyLeuArgValGlyTyrLys

         970                 990                1010
TTAAATGAATATTTAACATATAAGAATAAAGTGAAAAGAAGCATAAATATTATTAATATT
LeuAsnGluTyrLeuThrTyrLysAsnLysValLysArgSerIleAsnIleIleAsnIle
```

TABELLE 16:

```
        1030                 1050                 1070
TTAACATTCATATCTAAACATGTGTGGAAATATTTATTTCAGCATTCATCTGATTTACTT
LeuThrPheIleSerLysHisValTrpLysTyrLeuPheGlnHisSerSerAspLeuLeu

        1090                 1110                 1130
AAATCTCAAGATAGTATTTATGAGTATATGATATGTGATAAAAATATTTTATTAAATAAA
LysSerGlnAspSerIleTyrGluTyrMetIleCysAspLysAsnIleLeuLeuAsnLys

        1150                 1170                 1190
TTTATAAATGTACCAAAAGATTATGGAAATATAAATTGTGCTGCCTTTGCAGCAGGTATT
PheIleAsnValProLysAspTyrGlyAsnIleAsnCysAlaAlaPheAlaAlaGlyIle

        1210                 1230
GTTGAAGGCTTCCTCTGTAGTTCTGAATTC
ValGluGlyPheLeuCysSerSerGluPhe
```

TABELLE 17:

Nukleotidsequenz und abgeleitete Aminosäuresequenz des
3'Endes des P.falciparum (Isolat FCBR) Genes, das durch das
Insert des Klones 41-2 spezifiziert wird.

```
              1240               1260               1280
GAATTCCAAGCAGATGTTACAGCGCACACTATTCATGAAGGCGATGATAATTATAACACT
GluPheGlnAlaAspValThrAlaHisThrIleHisGluGlyAspAspAsnTyrAsnThr

              1300               1320               1340
ACTATTTTTATTAAATTTTATCCGGAAGTAGTGGAAAGAGAAAAAAACCACTAGATATTC
ThrIlePheIleLysPheTyrProGluValValGluArgGluLysAsnHis

              1360               1380               1400
ATATAAGGGTCACACAATAAATATATATATAATACATGTTGTATAAGTTGTCAAAAAA

              1420               1440               1460
TTTATATGGAAAAAAATAAATTAAATATGTAAATATATATATATATATATATATATAT

              1480               1500               1520
TCTTTCTTTCTTTCTTTTTTTTTTTTTTTTGTTATTATTAATGTATTATTTATCCTTATG

              1540               1560               1580
CATGGGATTATTTAACAAATTTATTGATAAAATAAATGTACCCCTTTTTTTTTTTTCTTT

              1600               1620               1640
TTTTTCTTTTTTTTTTTTTTGTATAAACATATTTATATATATTTATATTTAATTAAACCT

              1660               1680               1700
TTTTTAACATTTTAAATCTATATGAAATAATAAATGAAGACATGACTATTTTAATACAAG

              1720               1740               1760
GAAATTAATGGTTCCTTAAATTTCACATAAAAAAAAAAAATAAAACATATAATATATATAT

              1780               1800               1820
ATATATATATAAAACACTTGGTTCTAATTTTTTTTTTTTTTTTTTTTTTTTTTTTTTTAA

              1840               1860               1880
TTTGTATGGAGATATTATAATATTTAAACAATATATATGACATATATAGAGGACATACTG

              1900               1920               1940
TTACCAATATTTTCAATACATTGTTGGAATTTTTTATTTTTTCATATATCATACATAAGA

              1960               1980               2000
CCTTCTGGAAAAGAAAAAAGTAATAAAGTGTCTTATATACTATTAATTTTGAATATAGAT

              2020               2040               2060
TTTTTTTCTTTTCTTTCAAAATTAAAAAGTATTCTATCAATGTATGTAAAATATATAATT

              2080               2100               2120
TTACTTTTTTTTTGTTCTTTTTTCTATTTTTAAATACGTATGTCCTCGTTTTTTTTTTTT

              2140               2160               2180
CTTTTTATAACATTATTTTGCATATTCCAAATTTTTTCTATGTGTCCAAAAAAAAAAAAA

              2200               2220               2240
AAAAAAATAAAGTGTTAATAAAAAAATTAAATAATATATGTGAAGATACTTTTTTTAATA

              2260
TGCATATGTATATATATTTATATATATAGATC
```

**Patentansprüche**

**Patentansprüche für folgende Vertragsstaaten : AT, BE, CH, DE, FR, GB, IT, LI, LU, NL, SE**

1. Proteine von Plasmodium falciparum mit Aminosäuresequenzen nach Tabelle 1 bis Tabelle 17 und antigenwirksame Teilsequenzen davon.

2. Proteine nach Anspruch 1, gekennzeichnet durch Expression der Sequenzen der Klone 41-1, 41-2, 41-3, 41-4, 41-5, 41-6, 41-7, 41-8, 41-9, 41-10, 41-12, 41-13, 41-14, 41-15, 41-17 und 41-2gen.

3. Fusionsproteine, die Proteine nach Anspruch 1 enthalten.

4. DNA und RNA, codierend für Proteine nach Anspruch 1.

5. Gereinigte und isolierte DNA Sequenzen nach Tabelle 1 bis Tabelle 17, codierend für Proteine nach Anspruch 1, sowie dazu komplementäre und hybridisierende Sequenzen.

6. Vektoren und DNA-Strukturen, die für Proteine nach Anspruch 1 oder 2 oder Fusionsproteine nach Anspruch 3 codieren.

7. Vektoren oder DNA-Strukturen, die DNA-Sequenzen nach Anspruch 5 enthalten.

8. Wirtszellen, enthaltend DNA nach Anspruch 4 oder 5 oder einen Vektor nach Anpruch 6 oder 7.

9. Proteine nach Anspruch 1 - 3, exprimiert von einer Wirtszelle nach Anspruch 8.

10. Verfahren zur Herstellung von Proteinen nach Anspruch 1 oder 2, dadurch gekennzeichnet, daß die für die Proteine nach Tabelle 1 bis Tabelle 17 kodierenden DNA-Sequenzen in pro- oder eukaryotischen Wirtssystemen exprimiert werden.

11. Vaccine, enthaltend eines oder mehrere der Antigene nach Anspruch 1, 2 oder 3.

12. Vaccine, enthaltend Proteine oder Teile davon, die durch Expression der Klone 41-1, 41-2 und 41-3 erhalten werden.

13. Diagnostika, enthaltend DNA oder RNA nach Anspruch 4, 5, 6 oder 7.

14. Diagnostika, enthaltend Proteine nach Anspruch 1, 2 oder 3.

**Patentansprüche für folgende Vertragsstaaten : ES, GR**

1. Verfahren zur Herstellung von Plasmodium falciparum Proteinen, dadurch gekennzeichnet, daß die für die Aminosäuresequenzen oder antigen wirkenden Teile davon nach Tabelle 1 bis Tabelle 17 kodierenden DNA-Sequenzen in pro- oder eukaryotischen Wirtssystemen exprimiert werden.

2. Verfahren nach Anspruch 1, dadurch gekennzeichnet, daß die Sequenzen der Klone 41-1, 41-2, 41-3, 41-4, 41-5, 41-6, 41-7, 41-8, 41-9, 41-10, 41-12, 41-13, 41-14, 41-15, 41-17, 41-2 gen eingesetzt werden.

3. Verfahren nach Anspruch 1 oder 2, dadurch gekennzeichnet, daß durch Anligieren an Sequenzen der Klone nach Anspruch 2 von anderen Proteinen oder Teilen davon Fusionsproteine hergestellt werden.

4. Verfahren zur Herstellung von poly- oder monoklonalen Antikörpern, dadurch gekennzeichnet, daß die nach Anspruch 1 oder 2 hergestellten Plasmodium falciparum Proteine zur Immunisierung eingesetzt werden.

5. Verfahren zur Herstellung einer Vaccine, dadurch gekennzeichnet, daß eines oder mehrere der nach Anspruch 1 oder 2 hergestellten Antigene eingesetzt werden.

6. Verfahren zur Isolierung von Plasmodium falciparum Proteinen, dadurch gekennzeichnet, daß die nach Anspruch 4 hergestellten Antikörper eingesetzt werden.

7. Verfahren zur Herstellung von Diagnostika, dadurch gekennzeichnet, daß nach Anspruch 4 hergestellte

21

Antikörper eingesetzt werden.

**8.** Verfahren zur Herstellung von Diagnostika, dadurch gekennzeichnet, daß nach Anspruch 1 oder 2 erzeugte Plasmodium falciparum Proteine eingesetzt werden.

**9.** Verfahren zur Herstellung von Diagnostika, dadurch gekennzeichnet, daß die für Aminosäuresequenzen von Tabelle 1 bis Tabelle 17 kodierenden Nukleinsäuren eingesetzt werden.

**Claims**

**Claims for the following Contracting States : AT, BE, CH, DE, FR, GB, IT, LI, LU, NL, SE**

**1.** Proteins of Plasmodium falciparum with amino acid sequences shown in Table 1 to Table 17, and partial sequences thereof having antigenic activity.

**2.** Proteins as claimed in claim 1, characterized by expression of the sequences of the clones 41-1, 41-2, 41-3, 41-4, 41-5, 41-6, 41-7, 41-8, 41-9, 41-10, 41-12, 41-13, 41-14, 41-15, 41-17 and 41-2gen.

**3.** Fusion proteins which contain proteins as claimed in claim 1.

**4.** DNA and RNA coding for proteins as claimed in claim 1.

**5.** Purified and isolated DNA sequences shown in Table 1 to Table 17, coding for proteins as claimed in claim 1, as well as sequences complementary and hybridizing therewith.

**6.** Vectors and DNA structures which code for proteins as claimed in claim 1 or 2 or fusion proteins as claimed in claim 3.

**7.** Vectors or DNA structures which contain DNA sequences as claimed in claim 5.

**8.** Host cells containing DNA as claimed in claim 4 or 5 or a vector as claimed in claim 6 or 7.

**9.** Proteins as claimed in claim 1-3 expressed by a host cell as claimed in claim 8.

**10.** A process for the preparation of proteins as claimed in claim 1 or 2, which comprises expression, in pro-or eukaryotic host systems, of DNA sequences coding for the proteins shown in Table 1 to Table 17.

**11.** A vaccine containing one or more of the antigens as claimed in claim 1, 2 or 3.

**12.** A vaccine containing proteins or parts thereof, which are obtained by expression of the clones 41-1, 41-2 and 41-3.

**13.** A diagnostic aid containing DNA or RNA as claimed in claim 4, 5, 6 or 7.

**14.** A diagnostic aid containing proteins as claimed in claim 1, 2 or 3.

**Claims for the following Contracting States : ES, GR**

**1.** A process for the preparation of Plasmodium falciparum proteins, which comprises expression, in pro-or eukaryotic host systems, of DNA sequences coding for the amino acid sequences or parts thereof having antigen activity shown in Table 1 to Table 17.

**2.** The process as claimed in claim 1, wherein the sequences of the clones 41-1, 41-2, 41-3, 41-4, 41-5, 41-6, 41-7, 41-8, 41-9, 41-10, 41-12, 41-13, 41-14, 41-15, 41-17 and 41-2gen are used.

**3.** The process as claimed in claim 1 or 2, wherein fusion proteins are prepared by the ligation onto sequences of the clones as claimed in claim 2 of other proteins or parts thereof.

4. A process for the preparation of poly- or monoclonal antibodies, which comprises using the Plasmodium falciparum proteins prepared as claimed in claim 1 or 2 for the immunization.

5. A process for the preparation of a vaccine, which comprises using one or more of the antigens prepared as claimed in claim 1 or 2.

6. A process for the isolation of Plasmodium falciparum proteins, which comprises using the antibodies prepared as claimed in claim 4.

7. A process for the preparation of diagnostic aids, which comprises using antibodies prepared as claimed in claim 4.

8. A process for the preparation of diagnostic aids, which comprises using Plasmodium falciparum proteins produced as claimed in claim 1 or 2.

9. A process for the preparation of diagnostic aids, which comprises using the nucleic acids coding for amino acid sequences of Table 1 to Table 17.

**Revendications**

**Revendications pour les Etats contractants suivants : AT, BE, CH, DE, FR, GB, IT, LI, LU, NL, SE**

1. Protéines de *Plasmodium falciparum* à séquences d'aminoacides selon les tableaux 1 à 17 et séquences partielles à action antigénique de celles-ci.

2. Protéines selon la revendication 1, caractérisées par l'expression des séquences des clones 41-1, 41-2, 41-3, 41-4, 41-5, 41-6, 41-7, 41-8, 41-9, 41-10, 41-12, 41-13, 41-14, 41-15, 41-17 et 41-2-gène.

3. Protéines de fusion qui contiennent des protéines selon la revendication 1.

4. ADN et ARN codant pour des protéines selon la revendication 1.

5. Séquence d'ADN purifiée et isolée selon les tableaux 1 à 17, codant pour les protéines selon la revendication 1, ainsi que séquences complémentaires de celles-ci et s'hybridant.

6. Vecteurs et structures d'ADN codant pour des protéines selon la revendication 1 ou 2 ou des protéines de fusion selon la revendication 3.

7. Vecteurs ou structures d'ADN contenant des séquences d'ADN selon la revendication 5.

8. Cellules hôtes contenant de l'ADN selon la revendication 4 ou 5 ou un vecteur selon la revendication 6 ou 7.

9. Protéines selon les revendications 1 à 3, exprimées par une cellule hôte selon la revendication 8.

10. Procédé pour la production de protéines selon la revendication 1 ou 2, caractérisé en ce que les séquences d'ADN codant pour les protéines selon les tableaux 1 à 17 sont exprimées dans des systèmes hôtes procaryotes ou eucaryotes.

11. Vaccin contenant un ou plusieurs des antigènes selon la revendication 1, 2 ou 3.

12. Vaccin contenant des protéines ou des parties de celles-ci, qui sont obtenues par expression des clones 41-1, 41-2 et 41-3.

13. Agents de diagnostic contenant de l'ADN ou de l'ARN selon la revendication 4, 5, 6 ou 7.

14. Agents de diagnostic contenant des protéines selon la revendication 1, 2 ou 3.

**Revendications pour les Etats contractants : ES, GR**

# EP 0 322 712 B1

1. Procédé pour la production de protéines de *Plasmodium falciparum,* caractérisé en ce que les séquences d'ADN codant pour les séquences d'aminoacides ou des parties à action antigénique de celles-ci, selon les tableaux 1 à 17, sont exprimées dans des systèmes hôtes procaryotes ou eucaryotes.

2. Procédé selon la revendication 1, caractérisé en ce que l'on utilise les séquences des clones 41-1, 41-2, 41-3, 41-4, 41-5, 41-6, 41-7, 41-8, 41-9, 41-10, 41-12, 41-13, 41-14, 41-15, 41-17 et 41-2-gène.

3. Procédé selon la revendication 1 ou 2, caractérisé en ce que l'on produit des protéines de fusion par soudure d'autres protéines ou de parties de celles-ci à des séquences des clones selon la revendication 2.

4. Procédé pour la production d'anticorps poly- ou monoclonaux, caractérisé en ce que l'on utilise pour l'immunisation les protéines de *Plasmodium falciparum* produites selon la revendication 1 ou 2.

5. Procédé pour la préparation d'un vaccin, caractérisé en ce que l'on utilise un ou plusieurs des antigènes produits selon la revendication 1 ou 2.

6. Procédé pour l'isolement de protéines de *Plasmodium falciparum,* caractérisé en ce que l'on utilise les anticorps produits selon la revendication 4.

7. Procédé pour la préparation d'agents de diagnostic, caractérisé en ce que l'on utilise des anticorps produits selon la revendication 4.

8. Procédé pour la préparation d'agents de diagnostic, caractérisé en ce que l'on utilise des protéines de *Plasmodium falciparum* produites selon la revendication 1 ou 2.

9. Procédé pour la préparation d'agents de diagnostic, caractérisé en ce que l'on utilise les acides nucléiques codant pour des séquences d'aminoacides des tableaux 1 à 17.

24

FIG. 1 A

FIG. 1 B

EP 0 322 712 B1